# EUROPÄISCHE PATENTSCHRIFT

(11) **EP 2 116 232 B1**
(45) Veröffentlichungstag und Bekanntmachung des Hinweises auf die Patenterteilung: **15.02.2012**
(21) Anmeldenummer: 08008779.4
(22) Anmeldetag: 09.05.2008
(51) Int. Cl.: A61K 9/16

(54) **Galanthaminhaltiges Arzneimittel mit kontrollierter Freisetzung**
Medicine containing galanthamine with controlled release
Médicament à libération contrôlée comprenant de la galanthamine

(43) Veröffentlichungstag der Anmeldung: 11.11.2009
(73) Patentinhaber: Ratiopharm GmbH, 89079 Ulm (DE)
(72) Erfinder: Muskulus, Frank, 82194 Gröbenzell (DE); Pätz, Jana, 86424 Dinkelscherben (DE)
(74) Vertreter: Best, Michael

(56) Entgegenhaltungen:
- WO-A-00/38686
- WO-A-2005/065661
- WO-A-2008/048469
- US-A1- 2006 093 671
- LI Y-H ET AL: "Modulation of combined-release behaviors from a novel ''tablets-in-capsule system''" JOURNAL OF CONTROLLED RELEASE, ELSEVIER, AMSTERDAM, NL, Bd. 95, Nr. 3, 24. März 2004 (2004-03-24), Seiten 381-389, XP004496378 ISSN: 0168-3659

## Beschreibung

Die vorliegende Erfindung betrifft ein Arzneimittel mit kontrollierter Freisetzung. Das Arzneimittel enthält als Wirkstoff Galanthamin. Die Erfindung betrifft ebenfalls ein Verfahren zur Herstellung eines derartigen Arzneimittels und die Verwendung von Galanthamin zur Herstellung eines derartigen Arzneimittels zur Behandlung der Alzheimer-Erkrankung.

Galanthamin ist ein tertiäres Pflanzenalkaloid, welches aus dem kleinen Schneeglöckchen (Galanthus nivalis), dem Kaukasischen Schneeglöckchen (Galanthus woronovii) sowie aus einigen Narzissenarten wie etwa der Osterglocke gewonnen werden kann. Heute kann der Wirkstoff auch auf synthetischem Wege herstellt werden.

Die chemische Bezeichnung des Galanthamins ist [4aS-(4aα,6β,8aR)]-4a,5,9,10,11,12-Hexahydro-3-methoxy-11-methyl-6H-benzofuro[3a,3,2-ef][2]benzazepin-6-ol. Galanthamin wird in Arzneimitteln in der Regel als Säureadditionssalz eingesetzt, insbesondere als Hydrobromid. Sowohl Galanthamin selbst als auch sein Hydrobromid sind linksdrehend. Galanthamin ist als Acetylcholinesteraseinhibitor, der an nikotinischen Rezeptoren, nicht jedoch an muskarinischen Rezeptoren wirkt, bekannt. Es ist weiterhin dafür bekannt, die Blut-Hirn-Schranke im Menschen zu überwinden und weist daneben bei therapeutisch wirksamen Dosen wenig ernste Nebenwirkungen auf.

Anfangs wurde Galanthamin als Wirkstoff für die Aufhebung der durch Curare-Verbindungen ausgelösten Muskelentspannungen bei Operationen eingesetzt. Heute wird Galanthamin vorwiegend als Antidementivum zur Behandlung von Demenzen, insbesondere von Alzheimer eingesetzt.

WO 97/47304 offenbart schnell auflösende Galanthamintabletten bzw. Galanthamintabletten mit sofortiger Freisetzung, die durch direktes Verpressen hergestellt werden. Diese und andere im Stand der Technik bekannten Tabletten mit sofortiger Freisetzung werden zweimal oder dreimal täglich in Abständen von 8 Stunden verabreicht. Die Plasmaspiegel des Wirkstoffes steigen typischerweise sehr schnell an und nehmen sehr schnell wieder ab.

Die Nebenwirkungen des Galanthamins, insbesondere Übelkeit, Erbrechen, Schwitzen, Ruhelosigkeit und Schlaflosigkeit treten besonders bei Schwankungen und Sprüngen in der im Blut vorhandenen Wirkstoffkonzentration auf. Eine optimale Therapie mit Galanthamin besteht somit darin, dass die effektive Plasmakonzentration an Galanthamin möglichst konstant, d.h. tagsüber gleichmäßig hoch und nachts leicht abgesenkt konstant vorhanden bleibt.

Neuere Entwicklungen stellen daher Galanthaminarzneimittel mit kontrollierter, das heißt verzögerter Freisetzung des Wirkstoffs zur Verfügung, durch die über einen längeren Zeitraum eine weitgehend konstante Wirkstofffreisetzung erzielt werden soll. Derartige Galanthaminarzneimittel sind beispielsweise in der WO 00/38686 beschrieben. Die verzögerte Freisetzung wird durch eine die Freisetzung kontrollierende Membranbeschichtung kontrolliert, die auf der Wirkstoffschicht eines Teilchens aufgebracht ist. Auf diese Membranbeschichtung kann nochmals eine schnellfreisetzende Wirkstoffschicht aufgebracht werden, um die Freisetzung des Wirkstoffs in der ersten Stunde nach der Verabreichung zu erhöhen.

Die WO 05/065661 beschreibt Galanthaminarzneimittel, bei denen die Freisetzung im Wesentlichen über Acrylatpolymere eingestellt wird und die verschiedene Freisetzungsprofile aufweisen können. Bei einer Ausführungsform erfolgt eine sehr schnelle anfängliche Freisetzung von mehr als 75% des Wirkstoffs innerhalb einer halben Stunde, bei einer anderen Ausführungsform ist die Freisetzung des Galanthamins sehr langsam, so dass innerhalb der ersten Stunde weniger als 18% des Wirkstoffs freigesetzt werden.

Weiterhin wird in der WO07/029081 ein freisetzungsgesteuertes Arzneimittel beschrieben, welches aus einem Kern enthaltend Galanthamin und wasserunlösliche Hilfsstoffe besteht, wobei die gesteuerte Freisetzung durch eine freisetzungsbestimmende Schicht gewährleistet wird.

Es besteht Bedarf nach weiteren Galanthaminarzneimitteln mit verzögerter Wirkstofffreisetzung, insbesondere nach Arzneimitteln, die eine sehr schnelle anfängliche Freisetzung mit einer langandauernden Freisetzung des Wirkstoffs kombinieren.

Das Arzneimittel soll bevorzugt Blutspiegelwirkstoffspitzen sowie auch das sogenannte "dose dumping" verhindern. Es soll bei möglichst geringen Nebenwirkungen reproduzierbare Blutspiegelwerte erzeugen, dabei aber geringe Produktionskosten aufweisen und möglichst geringe Lösemittelrückstände haben.

Die Aufgabe der vorliegenden Erfindung ist daher, ein Arzneimittel mit kontrollierter Freisetzung von Galanthamin zur Verfügung zu stellen, das ein Freisetzungsprofil mit schneller Anfangsfreisetzung und gleichmäßig über einen längeren Zeitraum anhaltender Langzeitfreisetzung aufweist und das darüber hinaus die vorstehenden Vorteile zeigt.

Das Freisetzungsprofil soll bevorzugt derart sein, dass bereits nach 10 Minuten 15 bis 35% des Wirkstoffs freigesetzt sind, nach 2 Stunden aber erst 35 bis 45% und nach 6 Stunden bereits über 60% des Wirkstoffs der Formulierung freigesetzt sind.

Die Freisetzung wird, soweit nichts anderes angegeben ist, nach den Vorschriften der USP711 unter Verwendung einer Paddle-Vorrichtung Nr. 2 bei 37°C ± 0,5°C in den ersten 2 Stunden in 375 ml 0,1 M HCl mit anschließenden Umpuffern mit 125 ml USP-Puffer (Phosphatpuffer) bei pH 6,8 bestimmt (Rührgeschwindigkeit 50 UpM).

Diese Aufgabe wird durch den Gegenstand der Patentansprüche gelöst. Die Erfindung stellt daher Arzneimittel zur oralen Verabreichung des Wirkstoffs Galanthamin zur Verfügung, die zum einen Pellets aber auch mindestens eine Tablette aufweisen. Die Tablette setzt den Wirkstoff schnell frei, also nicht verzögert, und ist nicht beschichtet, die Pellets setzen den Wirkstoff verzögert frei, und durch die Kombination dieser beiden Arzneiformen, den das Galanthamin verzögert freisetzenden Pellets und der das Galanthamin schnell freisetzenden Tablette, wird das erfindungsgemäße, vorteilhafte Freisetzungsprofil erreicht. Die Kombination enthält weiterhin nur sehr geringe Mengen Restlösemittel, da ein Teil (die schnell freisetzende Tablette) lösemittelfrei durch Direktverpressen hergestellt werden kann. Es ist überraschend, dass durch ein derart einfaches und preiswert herzustellendes Arzneimittel das erfindungsgemäß gewünschte, vorteilhafte Freisetzungsprofil erzielt werden kann.

Die Pellets weisen ausschließlich einen inerten Kern und nur eine wirkstoffhaltige Beschichtung auf, die direkt auf dem inerten Kern angebracht ist.

Die Pellets setzen den Wirkstoff verzögert frei und sind entsprechend ausgestaltet. Die verzögerte Freisetzung des Wirkstoffs erfolgt, indem der Wirkstoff mit einem retardierenden Polymer als Beschichtung auf dem inerten Pelletkern aufgebracht wird. Die Pellets werden auf übliche Art und Weise durch an sich bekannte Beschichtungsverfahren hergestellt.

Als inerte Kerne für die Pellets kann jedes pharmazeutisch unbedenkliche inerte, vorzugsweise geschmacksneutrale Material verwendet werden, wie Zuckerkerne. Bevorzugt sind aber inerte wasserunlösliche Kerne aus mikrokristalliner Cellulose. Die Kerne sind kommerziell in verschiedenen Kornfraktionen erhältlich. Erfindungsgemäß kann jede Kerngröße verwendet werden, vorzugsweise Kerne mit Durchmessern von 100 bis 1400 µm, mehr bevorzugt 400 bis 1000 µm, insbesondere 500 bis 750 µm, z.B. etwa 500 µm. Mikrokristalline Cellulose ist besonders aufgrund fehlender Wasserlöslichkeit, geringer Friabilität (= Abrieb) und konstanter Rundheit bevorzugt. Des Weiteren werden durch den inerten Charakter der mikrokristallinen Cellulose unerwünschte Wechselwirkungen mit dem Wirkstoff vermieden.

Als inerte Kerne in den Pellets können neben den oben bevorzugt genannten weitere verschiedene Materialien eingesetzt werden, vorausgesetzt diese Materialien sind pharmazeutisch unbedenklich und weisen geeignete Dimensionen und Festigkeiten auf.

Auf den inerten Kern ist in den Pellets der erfindungsgemäßen Arzneimitteln die wirkstoffhaltige Schicht direkt aufgebracht. Durch die Schicht wird die verzögerte Freisetzung des Wirkstoffs gesteuert. Die Pellets enthalten damit keine weiteren Membranschichten, die für die verzögerte Freisetzung des Wirkstoffs ursächlich sind.

Die wirkstoffhaltige Schicht in den Pellets der erfindungsgemäßen Arzneimittel weist ein wasserunlösliches, filmbildendes Polymer auf. Als geeignete wasserunlösliche, filmbildende Polymere kann z.B. ein Celluloseether wie Ethylcellulose, ein Celluloseester wie Celluloseacetat oder ein Polyvinylalkohol genannt werden. Besonders bevorzugt ist Ethylcellulose. Neben dem wasserunlöslichen Polymer, wie Ethylcellulose, können die Beschichtungen gegebenenfalls weitere übliche Bestandteile von Retardbeschichtungen enthalten. Die Beschichtung kann noch zur Einstellung der Freisetzung, wie es im Stand der Technik bekannt ist, einen wasserlöslichen Hilfsstoff als Porenbildner aufweisen. Bevorzugt wird die Freisetzung aber nicht durch einen wasserlöslichen Hilfsstoff als Porenbildner eingestellt, sondern diese Funktion wird durch den Wirkstoff, das Galanthamin selbst, wahrgenommen. Erfindungsgemäß bevorzugt enthält die wirkstoffhaltige Beschichtung in den Pellets daher keine wasserlöslichen Porenbildner als Zusatzstoff.

Als Weichmacher in den Beschichtungsformulierungen können alle gängigen, pharmazeutisch unbedenklichen Weichmacher für wasserunlösliche Polymere, wie Ethylcellulose, verwendet werden. Geeignet sind z.B. Triethylcitrat oder Polyethylenglykol, am meisten bevorzugt ist Triethylcitrat. Der Weichmacher wird in den Dispersionen zum Beschichten der inerten Kerne in einer Menge von vorzugsweise 0,5 bis 10 Gew.-%, insbesondere von 1 bis 7 Gew.-% eingesetzt, jeweils bezogen auf das Gesamtgewicht der wässrigen Dispersion (z.B. 5 bis 30 Gew.-%, insbesondere 5 bis 20 Gew.-%, wie 7 bis 15 Gew.-%, bezogen auf das Gewicht der Wirkstoffschicht).

Neben den oben genannten Inhaltsstoffen können die erfindungsgemäßen Beschichtungen noch verschiedene übliche Zusatzstoffe wie Verdickungsmittel, Schmiermittel, Tenside, Konservierungsstoffe, Komplexbildner, Chelatbildner und/oder Elektrolyte enthalten.

Die relativen Anteile der Bestandteile der Beschichtungen, insbesondere das Verhältnis von wasserunlöslichem, filmbildendem Polymer zu den anderen Bestandteilen, bestimmen das Freisetzungsprofil der Beschichtung.

Besonders bevorzugt als Beschichtungsmaterial ist erfindungsgemäß eine Mischung aus Ethylcellulose, einem Tensid wie Natriumlaurylsulfat und Cetylalkohol (z.B. eine solche Mischung, wie sie als AQUACOAT™ kommerziell erhältlich ist) mit Triethylcitrat als Weichmacher.

Die inerten Kerne werden zur Herstellung der Pellets vorzugsweise mit wässrigen Dispersionen der Beschichtungsmaterialien, in die der Wirkstoff eingemischt wird, beschichtet.

Erfindungsgemäß bestehen die Pellets ausschließlich aus einem inerten Kern und der darauf angebrachten einzigen wirkstoffhaltigen Schicht, die den Wirkstoff retardiert freisetzt.

Die Pellets des Arzneimittels der vorliegenden Erfindung lassen sich auf übliche Art und Weise herstellen. Die Beschichtung kann auf wässrige oder nicht-wässrige Art und Weise erfolgen. Bei einer wässrigen Verfahrensführung, die erfindungsgemäß bevorzugt ist, wird z.B. eine entsprechende Menge an Galanthamin oder ein pharmazeutisch verträgliches Salz oder Solvat davon in einer geeigneten Menge Wasser aufgelöst oder dispergiert. Hierzu werden die weiteren Bestandteile der Beschichtung, z.B. der ggf. vorhandene Weichmacher zugesetzt. Das wasserunlösliche Polymer wird dann bevorzugt als wässrige Dispersion, die in der Regel noch zumindest ein Tensid enthält, mit der Lösung bzw. der Dispersion des Wirkstoffs vermischt. Die inerten Kerne werden mit dieser Dispersion beschichtet. Für eine nicht-wässrige Beschichtungsform können übliche Lösemittel wie Alkohole, z.B. Ethanol, eingesetzt werden. Die Menge an Wirkstoff in der das Arzneimittel enthaltenden Beschichtungslösung/Dispersion kann im Bereich von 0,1 bis 10 Gew.-% liegen, vorzugsweise 1 bis 5 Gew.-%, insbesondere 2 bis 3 Gew.-%, jeweils bezogen auf das Gesamtgewicht der Dispersion bzw. Lösung.

Das Beschichten der inerten Kerne kann z.B. in einem Wirbelschichtgranulator (z.B. Glatt Typ BSG-30 oder GPCG-30) durchgeführt werden, der vorzugsweise mit einem Wurster-Bottom-Spray-Einsatz (z.B. einem 18-Zoll-Wurster-Einsatz) ausgestattet ist.

Die erfindungsgemäßen Arzneimittel weisen neben den Pellets noch mindestens eine Tablette auf, die ebenfalls den Wirkstoff in Kombination mit üblichen Hilfs- und Zusatzstoffen enthält. Diese Tablette wird auf übliche Art und Weise durch Feuchtgranulation und Verpressen eines Granulats, vorzugsweise jedoch durch Direktverpressung, hergestellt. Diese wirkstoffhaltige Tablette setzt den Wirkstoff schnell frei. Die Tablette, die den Wirkstoff schnell freisetzt, ist nicht beschichtet. Die Erfindung wird im Folgenden am Beispiel der nicht beschichteten Tabletten erläutert.

Bevorzugt enthält das Arzneimittel 1 bis 5 derartige nicht beschichtete Tabletten, noch stärker bevorzugt 1 oder 2 nicht beschichtete Tabletten und am stärksten bevorzugt eine nicht beschichtete Tablette. Unter "Tablette", wie dieser Ausdruck in der vorliegenden Beschreibung und den Ansprüchen verwendet wird, fallen insbesondere die dem Fachmann bekannten sogenannten Minitabletten, deren Herstellung in den Beispielen beschrieben wird.

Die nicht beschichteten Tabletten in den Arzneimitteln der vorliegenden Erfindung setzen den Wirkstoff schnell frei, das heißt eine vollständige Freisetzung erfolgt innerhalb von höchstens 15 Minuten, bevorzugt innerhalb von höchstens 10 Minuten.

Die nicht beschichteten Tabletten in den erfindungsgemäßen Arzneimitteln weisen übliche Hilfs- und Zusatzstoffe auf, bevorzugt weisen sie ein Gemisch aus mindestens einem wasserlöslichen Zusatzstoff und mindestens einem wasserunlöslichen Zusatzstoff auf. Bei dem wasserlöslichen Zusatzstoff handelt es sich bevorzugt um ein Bindemittel, wobei übliche wasserlösliche Bindemittel wie z.B. das Produkt Povidon VA64 genannt werden können. Die nicht beschichteten Tabletten weisen weiterhin in der Regel auch ein oder mehrere Füllstoffe auf, wobei sie insbesondere auch wasserlösliche Füllstoffe enthalten können, wie Laktose und andere Zuckeralkohole.

Bei dem wasserunlöslichen Zusatzstoff, der bevorzugt Bestandteil der nicht beschichteten Tabletten der erfindungsgemäßen Arzneimittel ist, handelt es sich bevorzugt um ein Füllmittel, das bevorzugt zusammen mit einem wasserlöslichen Füllmittel vorliegen kann. Besonders bevorzugt als wasserunlösliches Füllmittel ist die mikrokristalline Cellulose, andere übliche Füllmittel sind beispielsweise anorganische Salze wie Calciumcarbonat und Calciumhydrogenphosphat.

Unter einem wasserlöslichen Zusatzstoff wird erfindungsgemäß ein Zusatzstoff verstanden, dessen Löslichkeit in Wasser mindestens 10 g pro 100 ml beträgt, unter einem wasserunlöslichen Zusatzstoff ein Zusatzstoff, dessen Löslichkeit in Wasser höchstens 1 g pro 100 ml beträgt.

Die nicht beschichteten Tabletten in den erfindungsgemäßen Arzneimitteln enthalten in der Regel auch weitere übliche Hilfs- und Zusatzstoffe, bevorzugt Sprengmittel, Schmiermittel, Geschmacksmittel, etc. Besonders bevorzugt enthalten sie ein nicht quervernetztes Sprengmittel. Ein bevorzugtes Schmiermittel ist Magnesiumstearat.

Die Herstellung der unbeschichteten Tabletten mit dem Wirkstoff Galanthamin erfolgt auf bekannte Art und Weise, bevorzugt erfolgt die Herstellung über ein Direktverpressungsverfahren. Hierzu können die Bestandteile in einer für die Direktverpressung geeigneten Qualität, beispielsweise in einem Freifallmischer (Turbola) gemischt und anschließend auf einer Rundläuferpresse zu Tabletten, insbesondere Minitabletten, verpresst werden.

Der Wirkstoff ist erfindungsgemäß Galanthamin, das als freie Base, bevorzugt aber in Form eines pharmazeutisch verträglichen Salzes, insbesondere in Form eines pharmazeutisch verträglichen Säureadditionssalzes vorliegt. Der Wirkstoff bzw. das Salz kann auch als Solvat, z.B. Hydrat, vorliegen. Wenn im Rahmen dieser Anmeldung von dem "Wirkstoff" oder "Galanthamin" gesprochen wird, ist hierunter der Wirkstoff in Form der freien Base oder in Form eines pharmazeutisch verträglichen Salzes, insbesondere eines pharmazeutisch verträglichen Säureadditionssalzes zu verstehen, wobei die freie Base oder das Salz auch in Form eines Solvats, insbesondere eines Hydrates vorliegen kann. Besonders bevorzugt liegt der Wirkstoff erfindungsgemäß als Galanthaminhydrobromid vor. Auch Gemische mehrerer Wirkstoffformen, z.B. Solvat und Nicht-Solvat, sind erfindungsgemäß erfasst.

Neben dem Wirkstoff Galanthamin (bzw. dem Salz oder Solvat davon) kann das erfindungsgemäße Arzneimittel noch von Galanthamin verschiedene Wirkstoffe enthalten, bevorzugt enthält das erfindungsgemäße Arzneimittel aber ausschließlich den Wirkstoff Galanthamin (bzw. ein Salz oder Solvat davon) und keinen weiteren Wirkstoff.

Das erfindungsgemäße Arzneimittel weist sowohl die das Galanthamin verzögert freisetzenden Pellets als auch die das Galanthamin schnell freisetzenden Tabletten auf. Das Arzneimittel kann entweder so ausgestaltet sein, dass die Pellets und die Tablette bzw. die Tabletten miteinander in einer Einheitsdosisform kombiniert werden oder dass die Pellets und die Tablette bzw. die Tabletten getrennt voneinander zu Einheitsdosisformen formuliert werden und dann mit einem Hinweis versehen werden, dass sie zur gleichzeitigen Einnahme vorgesehen sind. Bevorzugt handelt es sich bei dem Arzneimittel jedoch um eine Ausführungsform, bei der die Pellets und die Tablette bzw. die Tabletten zu einer Einheitsdosisform kombiniert werden, beispielsweise in einer Hartgelatinekapsel oder einem Sachet, bevorzugt einer Hartgelatinekapsel. Diese Hartgelatinekapsel enthält dann sowohl die Pellets als auch eine oder mehrere der unbeschichteten Tabletten.

Die Pellets und Tabletten können in automatischen Standart-Kapselfüllmaschinen in Hartgelatinekapseln abgefüllt werden.

Der Gehalt an Wirkstoff in dem erfindungsgemäßen Arzneimittel ist nicht besonders eingeschränkt. Bevorzugt weist eine Dosierungsform, das heißt insbesondere eine Hartgelatinekapsel, eine Menge von 1 bis 30 mg Wirkstoff auf, besonders bevorzugt eine Menge von 3 bis 25 mg, insbesondere eine Menge von 6 bis 24 mg. Bevorzugt ist der Anteil des Wirkstoffs in den Pellets höher als in der Tablette, bevorzugt finden sich 1,5 bis 6 mal soviel Wirkstoff in den Pellets wie in der Tablette. Bevorzugt enthalten die Pellets in einer Dosierungseinheit des erfindungsgemäßen Arzneimittels 3 bis 23 mg Wirkstoff, bevorzugt 4 bis 20 mg Wirkstoff. Die Tablette bzw. die Tabletten in einer Dosierungseinheit enthalten bevorzugt 1,5 bis 10 mg, stärker bevorzugt 1,5 bis 8 mg an Wirkstoff. Die Wirkstoffangaben im Rahmen dieser Anmeldung beziehen sich immer auf den Wirkstoff als freie Base, alle Angaben beziehen sich auf das Gewicht, soweit nichts anderes ausdrücklich angegeben oder aufgrund der Umstände ersichtlich ist.

Die erfindungsgemäßen Arzneimittel stellen ein besonders vorteilhaftes Freisetzungsprofil zur Verfügung, insbesondere ein Freisetzungsprofil, bei dem bereits nach 10 Minuten 15 bis 35% des Wirkstoffs des Arzneimittels freigesetzt sind, nach 2 Stunden 35 bis 45% des Wirkstoffs und nach 6 Stunden über 60% des Wirkstoffs des Arzneimittels freigesetzt sind.

Das Freisetzungsprofil kann durch Gehaltsänderung der Initialdosis oder durch die Wahl der Menge an wasserunlöslichem Polymer in der wirkstoffhaltigen Schicht der Pellets eingestellt werden.

Die Erfindung wird durch folgende Beispiel weiter veranschaulicht werden:

### Beispiele 1 bis 3

Pellets mit kontrollierter Freisetzung wurden hergestellt, indem das Galanthaminsalz in Wasser gelöst wird. Triethylcellulose wird mit Aquacoat gemischt und in die Galanthaminlösung eingerührt. Hierdurch entsteht eine Dispersion. Mit dieser Dispersion werden Kerne der Marke Cellet 500 in einem üblichen Wirbelschichtgerät beschichtet. Die Pellets werden auf übliche Art und Weise getrocknet. Die eingesetzten Mengen an Wirkstoff und Hilfsstoffen sind der folgenden Tabelle 1 zu entnehmen.

Unbeschichtete Tabletten mit schneller Freisetzung wurden hergestellt, indem die Bestandteile, das Galanthaminsalz, das Avicel, die Tablettose, das Kollidon VA64 und Explotab in einem Freifallmischer vorgemischt und im Anschluss mit Magnesiumstearat vermischt wurden. Die so hergestellte Fertigmischung wird auf einer Rundläuferpresse zu Minitabletten mit einem Radius von 2,8 mm verpresst. Auch hier sind die Mengenbestandteile in der folgenden Tabelle 1 angegeben.

Die Pellets und eine Tablette wurden jeweils in eine übliche Hartgelatinekapsel eingefüllt, so dass sich der in der Tabelle 1 angegebene Galanthamingehalt ergibt.

In den Beispielen 1 und 3 beträgt die Initialdosis 25%, in Beispiel 2 beträgt die Initialdosis 30%.

**Tabelle 1**

| | **Inhaltsstoffe** (Angaben in mg) | **Beispiel 1** | **Beispiel 2** | **Beispiel 3** |
|---|---|---|---|---|
| | **ER-Pellets** | | | |
| 1 | Galantamin entspricht | (6,0) | (5,6) | (6,0) |
| 2 | Galantamin HBr* | 7,8 | 7,2 | 7,8 |
| 3 | TEC | 6,1 | 5,7 | 5,6 |
| 4 | Aquacoat ECD (Trockenmasse) | 33 | 30,8 | 29,6 |
| 5 | Cellets 500 | 37,5 | 35,0 | 37,5 |

| | **IR-Tablette** | | | |
|---|---|---|---|---|
| 6 | Galantamin entspricht | (2,0) | (2,4) | (2,0) |
| 7 | Galanthamin HBr* | 2,6 | 3,1 | 2,6 |
| 8 | Avicel PH 102** | 11,4 | 13,6 | 11,4 |
| 9 | Tablettose 80 | 15,4 | 18,5 | 15,4 |
| 10 | Kollidon VA 64 | 1,6 | 1,9 | 1,6 |
| 11 | Explotab | 1,3 | 1,6 | 1,3 |
| 12 | Magnesiumstearat | 0,3 | 0,4 | 0,3 |
| | GESAMTMENGE | 117,0 | 117,8 | 113,1 |

| | | | | |
|---|---|---|---|---|
| *Galanthamin HBr wird gemäß Wirkstoffgehalt eingewogen. **Avicel PH 102 gilt als Ausgleichsubstanz | | | | |

### Testbeispiel 1 bis 3

Die Kapseln der Beispiele 1, 2 und 3 wurden nach den Vorschriften der USP 711 in einer Paddle-Vorrichtung Nr. 2 in den ersten beiden Stunden in 375 ml 0,1 M HCl mit anschließenden Umpuffern mit 125 ml USP-Puffer (Phosphatpuffer) bei pH 6,8 freigesetzt (Rührgeschwindigkeit 50 UpM).
Die Wirkstoffmenge betrug jeweils 8 mg Galanthamin als freie Base, es wurden die vollständigen Kapseln mit Pellets und Tabletten eingesetzt. Die Temperatur betrug 37°C ± 0,5°C. Es wurde bei einer Umdrehung des Paddles von 50 UpM gearbeitet. Die Küvetten hatten eine Dicke von 10 mm, die Messungen erfolgten bei einer Wellenlänge von 289 nm und einer Referenzwellenlänge von 400 bis 450 nm.

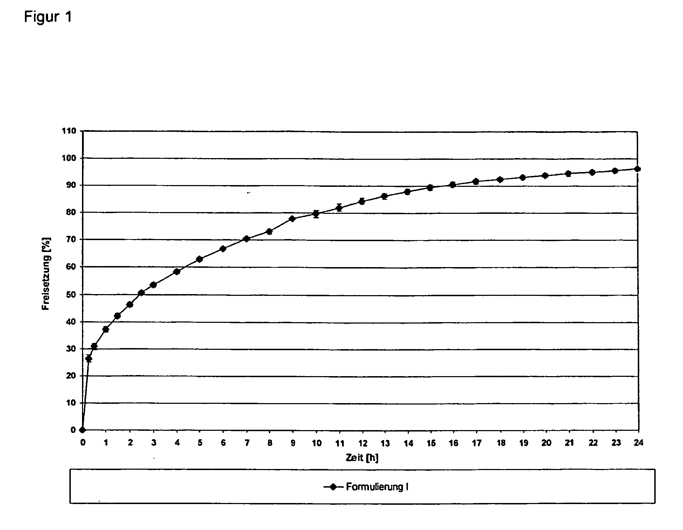

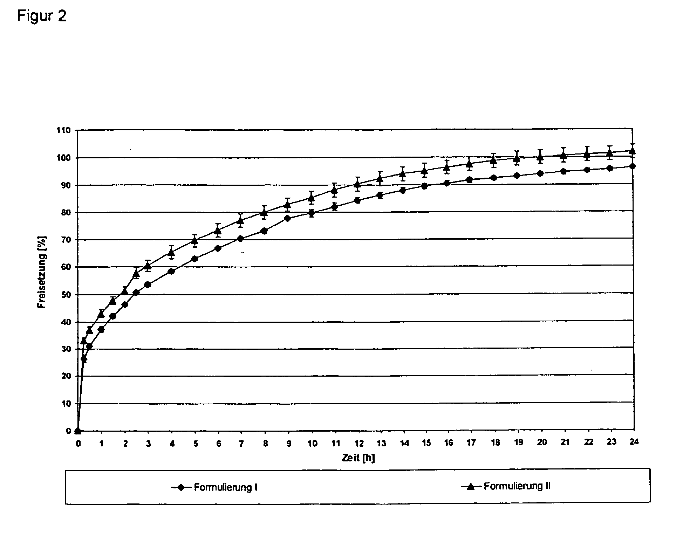

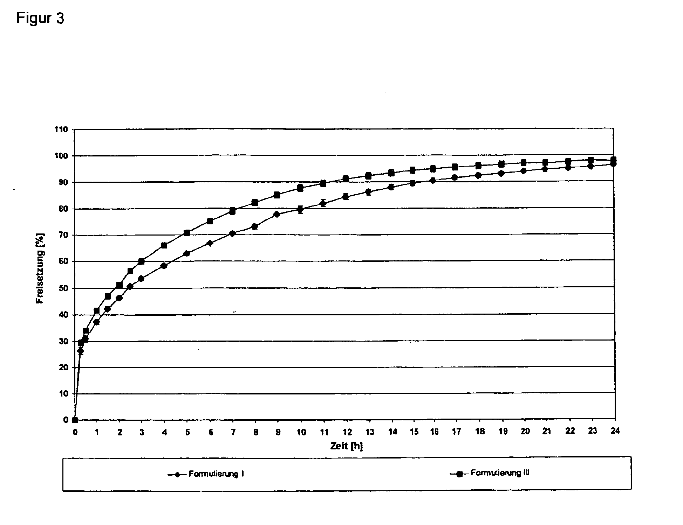

Die Ergebnisse der Freisetzungstests sind in den Figuren 1, 2 und 3 dargestellt. Figur 1 zeigt die Freisetzung des Arzneimittels des Beispiels 1 (Vierecke), Figur 2 zeigt die Freisetzung der Arzneimittel der Beispiele 1 (Rechtecke) und 2 (Dreiecke), und Figur 3 zeigt das Freisetzungsprofil des Arzneimittels des Beispiels 1 (untere Kurve) und des Beispiels 3 (obere Kurve).

Es zeigt sich, dass das Arzneimittel des Beispiels 2 und das Arzneimittel des Beispiels 3 den Wirkstoff schneller freisetzen als das Arzneimittel des Beispiels 1. Dies ist darauf zurückzuführen, dass in Beispiel 2 der Anteil des Wirkstoffs in der unbeschichteten Tablette erhöht ist und in den Beispielen 2 und 3 die Menge an unlöslichem Polymer in der Beschichtung der Pellets reduziert wurde.

In allen drei Beispielen ist die Freisetzung vorteilhaft.

## Patentansprüche

1. Arzneimittel zur oralen Verabreichung des Wirkstoffs Galanthamin, eines pharmazeutisch verträglichen Salzes oder eines Solvats davon, umfassend Pellets und mindestens eine nicht beschichtete Tablette, wobei die Pellets ausschließlich aus einem inerten Kern und der darauf aufgebrachten einzigen wirkstoffhaltige Beschichtung, die den Wirkstoff verzögert freisetzt, bestehen, wobei die wirkstoffhaltige Beschichtung der Pellets ein wasserunlösliches, filmbildendes Polymer enthält, und die nicht beschichtete Tablette den Wirkstoff nicht verzögert, das heißt vollständig innerhalb von höchstens 15 Minuten freisetzt.

2. Arzneimittel nach Anspruch 1, wobei die mindestens eine Tablette eine direkt verpresste Tablette ist.

3. Arzneimittel nach einem der Ansprüche 1 oder 2, wobei die mindestens eine Tablette mindestens einen wasserlöslichen Zusatzstoff und mindestens einen wasserunlöslichen Zusatzstoff enthält.

4. Arzneimittel nach Anspruch 3, wobei der wasserlösliche Zusatzstoff ein wasserlösliches Bindemittel darstellt.

5. Arzneimittel nach Anspruch 3 oder 4, wobei der wasserunlösliche Zusatzstoff mikrokristalline Cellulose ist.

6. Arzneimittel nach einem der Ansprüche 1 bis 5, wobei das Arzneimittel als Einheitsdosisform vorliegt.

7. Arzneimittel nach Anspruch 6, wobei die Einheitsdosisform eine Hartgelatinekapsel ist, die die Pellets und die mindestens eine Tablette enthält.

8. Arzneimittel nach Anspruch 6 oder 7, wobei die Einheitsdosisform 1,5 bis 25 mg Wirkstoff enthält.

9. Arzneimittel nach einem der Ansprüche 1 bis 8, wobei der Anteil des Wirkstoffs in den Pellets 1,5 bis 6 mal so hoch ist, wie der Anteil des Wirkstoffs in der Tablette (bezogen auf das Gewicht des Wirkstoffs).

10. Arzneimittel nach einem der Ansprüche 1 bis 9, **gekennzeichnet durch** folgendes Freisetzungsprofil:
10 bis 35 Gew.-% des Wirkstoffs werden innerhalb der ersten 10 Minuten freigesetzt,
35 bis 45 Gew.-% werden innerhalb der ersten 2 Stunden freigesetzt,
mehr als 60 Gew.-% werden innerhalb der ersten 6 Stunden freigesetzt.

11. Verfahren zur Herstellung eines Arzneimittels nach einem der Ansprüche 1 bis 10, bei dem
a) Pellets hergestellt werden, indem inerte Kerne mit der darauf aufgebrachten einzigen wirkstoffhaltigen Beschichtung versehen werden,
b) mindestens eine schnell freisetzende, wirkstoffhaltige Tablette hergestellt wird und
c) die Pellets aus Schritt a) mit der Tablette aus Schritt b) kombiniert werden.

12. Verwendung von Galanthamin zur Herstellung eines Arzneimittels wie in einem der Ansprüche 1 bis 10 definiert zur Behandlung der Alzheimer Erkrankung.

## Claims

1. A pharmaceutical preparation for oral administration of the active ingredient galanthamine, of a pharmaceutically well-tolerated salt or a solvate thereof, comprising pellets and at least one non-coated tablet, wherein the pellets solely consist of an inert core and the only active ingredient-containing coating applied thereto, which releases the active ingredient retarded, wherein the active ingredient-containing coating of the pellets contains a water-insoluble, film-forming polymer, and the non-coated tablet does not release the active ingredient retarded, i.e. releases it completely within at most 15 minutes.

2. The pharmaceutical preparation according to claim 1, wherein the at least one tablet is a directly compacted tablet.

3. The pharmaceutical preparation according to any one of claims 1 or 2, wherein the at least one tablet contains at least one water-soluble additive and at least one water-insoluble additive.

4. The pharmaceutical preparation according to claim 3, wherein the water-soluble additive represents a water-soluble binder.

5. The pharmaceutical preparation according to claim 3 or 4, wherein the water-insoluble additive is microcrystalline cellulose.

6. The pharmaceutical preparation according to any one of claims 1 to 5, wherein the pharmaceutical preparation is present in unit dosage form.

7. The pharmaceutical preparation according to claim 6, wherein the unit dosage form is a hard gelatin capsule containing the pellets and the at least one tablet.

8. The pharmaceutical preparation according to claim 6 or 7, wherein the unit dosage form contains 1.5 to 25 mg active ingredient.

9. The pharmaceutical preparation according to any one of claims 1 to 8, wherein the proportion of the active ingredient in the pellets is 1.5 to 6 times as high as the proportion of the active ingredient in the tablet (based on the weight of the active ingredient).

10. The pharmaceutical preparation according to any one of claims 1 to 9, **characterized by** the following releasing profile:
10 to 35 wt-% of the active ingredient are released within the first 10 minutes,
35 to 45 wt-% are released within the first 2 hours,
more than 60 wt-% are released within the first 6 hours.

11. A method for the preparation of a pharmaceutical preparation according to any one of claims 1 to 10, wherein
a) pellets are prepared by providing inert cores with the only active ingredient-containing coating applied thereto,
b) at least one fast-releasing, active ingredient-containing tablet is prepared, and
c) the pellets of step a) are combined with the tablet of step b).

12. Use of galanthamine for the preparation of a pharmaceutical preparation as defined in any one of claims 1 to 10 for the treatment of Alzheimer's disease.

## Revendications

1. Médicament pour administration orale de l'agent actif galanthamine, d'un sel ou d'un solvate acceptable du point de vue pharmaceutique de ce dernier, comprenant des pastilles et au moins un comprimé non revêtu, sachant que les pastilles se composent exclusivement d'un coeur inerte et d'un revêtement unique, contenant l'agent actif, appliqué sur ce dernier, qui libère avec retard l'agent actif, sachant que le revêtement, contenant l'agent actif, des pastilles contient un polymère filmogène non hydrosoluble et que le comprimé non revêtu ne retarde pas l'agent actif, c'est-à-dire que ce dernier est complètement libéré en l'espace d'une période d'au plus 15 minutes.

2. Médicament selon la revêtement 1, sachant que le au moins un comprimé est un comprimé directement pressé.

3. Médicament selon l'une quelconque des revendications 1 ou 2, sachant que le au moins un comprimé contient au moins un adjuvant hydrosoluble et au moins un adjuvant non hydrosoluble.

4. Médicament selon la revêtement 3, sachant que l'adjuvant hydrosoluble est constitué d'un liant hydrosoluble.

5. Médicament selon la revendication 3 ou 4, sachant que l'adjuvant non hydrosoluble est la cellulose microcristalline.

6. Médicament selon l'une quelconque des revendications 1 à 5, sachant que le médicament est présent sous la forme d'une dose unitaire.

7. Médicament selon la revêtement 6, sachant que la forme de dose unitaire est une capsule de gélatine dure, qui contient les pastilles et le au moins un comprimé.

8. Médicament selon la revendication 6 ou 7, sachant que la forme de dose unitaire contient de 1,5 à 2,5 mg d'agent actif.

9. Médicament selon l'une quelconque des revendications 1 à 8, sachant que la proportion de l'agent actif dans les pastilles est de 1,5 à 6 fois plus élevée que la proportion de l'agent actif dans le comprimé (par rapport au poids de l'agent actif).

10. Médicament selon l'une quelconque des revendications 1 à 9, **caractérisé par** le profil de libération suivant :
10 à 35 % en poids de l'agent actif sont libérés au cours des premières 10 minutes,
35 à 45 % en poids sont libérés au cours des premières 2 heures,
plus de 60 % en poids sont libérés au cours des premières 6 heures.

11. Procédé de fabrication d'un médicament selon l'une quelconque des revendications 1 à 10, lors duquel
a) les pastilles sont fabriquées, en enduisant les coeurs internes d'un revêtement unique, contenant l'agent actif, appliqué sur ces derniers,
b) au moins un comprimé contenant l'agent actif, à libération rapide est fabriqué, et
c) les pastilles issues de l'étape a) sont combinées au comprimé issu de l'étape b).

12. Utilisation de la galanthamine en vue de la fabrication d'un médicament tel que défini dans l'une des revendications 1 à 10 pour le traitement de la maladie d'Alzheimer.
